# EUROPEAN PATENT SPECIFICATION

(11) **EP 2 493 842 B1**
(45) Date of publication and mention of the grant of the patent: **28.03.2018**
(21) Application number: 10827208.9
(22) Date of filing: 29.10.2010
(51) Int. Cl.: C07C 69/67, A01P 19/00, A01N 37/06, C07C 67/08, A01N 37/36

(54) **CHRYSANTHEMYL DERIVATIVES AS MEALYBUG ATTRACTANT**
CHRYSANTHEMYLDERIVATE ALS LOCKMITTEL FÜR SCHMIERLÄUSE
DÉRIVÉS DE CHRYSANTHÉMYLE EN TANT QUE COMPOSÉ ATTIRANT LES COCHENILLES

(30) Priority: 30.10.2009 NZ 58085209
(43) Date of publication of application: 05.09.2012
(73) Proprietor: The New Zealand Institute for Plant and Food Research Limited, Auckland, 1025 (NZ)
(72) Inventor: El-Sayed, Ashraf M., Bromley, Christchurch, 8062 (NZ); Bergmann, Jan Heinrich Albrecht, Vina del Mar (CL); Uneluis, Carl Rikard, Lincoln, Christchurch 7608 (NZ)
(74) Representative: Kremer, Simon Mark
(86) International application number: PCT/NZ2010/000217
(87) International publication number: WO 2011/053168

(56) References cited:
- US-A- 4 521 331
- DATABASE REGISTRY STN 17 August 2006 XP008156472 Retrieved from STN Database accession no. 902267-72-5
- HO, H.-Y. ET AL.: 'Identification and synthesis of the sex pheromone of the Madeira mealybug, Phenacoccus Madeirensis Green' JOURNAL OF CHEMICAL ECOLOGY vol. 35, 2009, pages 724 - 732, XP008155163
- MILLAR, J. G. ET AL.: 'Chemistry and Application of Mealybug Pheromones' SEMIOCHEMICALS IN PEST AND WEED CONTROL 2005, pages 11 - 27, XP008155165
- EL-SAYED, A. M. ET AL.: 'Chysanthemyl 2-acetoxy-3-methylbutonate: the sex pheromone of the citrophilous mealybug, Pseudococcus calceolariae' TETRAHEDRON LETTERS vol. 51, 2010, pages 1075 - 1078, XP008155166

## Description

### 1. FIELD OF THE INVENTION

The invention relates to compounds for use as mealybug attractants, and to methods of controlling, and monitoring populations of mealybugs using the attractant compounds of the invention.

### 2. BACKGROUND OF THE INVENTION

Mealybugs (or woolly aphids) are scale insects that attack a range of plant species including economically important crops. They are small with a hairy, waxy covering that repels water. They reproduce rapidly, generally laying their eggs under a cotton-like covering. Mealybugs are polyphagous and may inject toxins and pathogens into the plant epidermis resulting in the spread of viruses.

Non-chemical methods of control include physically removing the insects from the plants, spraying them off with jets of water and pruning the infested plant. However, these methods are not suitable for use with large scale horticultural crops.

Insecticides based on organophosphorus compounds may be effective but the mealybugs' woolly, waxy coating is difficult for contact insecticides to penetrate. In addition, the insects often live deep inside cracks and crevices in the plant or inside fruit bunches where insecticides may not reach. To make matters worse, many strains of mealybug have built up resistance to chemical insecticides.

The Citrophilous mealybug, *Pseudococcus calceolariae* feeds on a variety of host plants including citrus, avocado, berries, sugarcane, cocoa, grape, and apple. In addition to malformation of fruits, shoots and leaves caused by injected toxins, the Citrophilous mealybug may also affect fruit quality by excreting honeydew which facilitates the development of fungi.

In New Zealand, the Citrophilous mealybug is a vector of grapevine leafroll-associated virus type 3 (GLRaV-3) (Petersen & Charles 1997), which causes significant declines in quantitative and qualitative parameters of vine performance. Due to quarantine restrictions imposed by several countries, the presence of the Citrophilous mealybug can cause significant economic losses to fruit exporters. HO et al identify and synthesise the sex pheromone of the Madeira mealybug, Phenacoccus Madeirensis Green (Journal of Chemical Ecology, 2009, Vol 35, pages 724-732).

As a result of rising global temperatures, insects including mealybugs are expanding their ranges into new habitats, increasing the economic losses caused by the insects. Therefore, effective methods of controlling and monitoring mealybugs are needed worldwide.

It is an object of the present invention to provide an effective method of mealybug control, or at least to provide the public with a useful choice.

### 3. SUMMARY OF THE INVENTION

Insect pheromones are volatile chemicals that insects use for mate location and other purposes critical to their lives.

Recently, researchers have found that mealybug pheromones may provide an economical and convenient means of detecting mealybug infestations and monitoring population changes. Mealybug pheromones strongly attract male mealybugs. This attraction can be used to lure the males into traps where they can be killed by contact with insecticides or other means. The pheromones can also be used to disrupt mealybug mating.

The pheromone for the Citrophilous mealybug has now been identified as chrysanthemyl 2-acetoxy-3-methylbutanoate (2,2-dimethyl-3-(2-methylprop-1-enyl)cyclopropyl)methyl 2-acetoxy-3-methylbutanoate). Consequently, this compound and its derivatives may be useful in methods of controlling and monitoring populations of mealybugs, particularly Citrophilous mealybugs.

In one aspect this disclosure provides a compound of formula I or a salt thereof 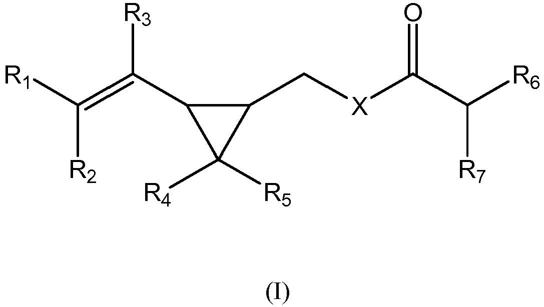 wherein R₁, R₂, R₃, R₄, R₅ and R₆ are independently selected from
- (C₁ - C₁₂)alkyl
- (C₂ - C₁₂)alkenyl,
- (C₂ - C₁₂)alkynyl,
- aryl,
- heteroaryl,
- aryl (C₁ - C₁₂)alkyl,
- heteroarylalkyl,
- heterocycle,
- heterocyclealkyl,
- halo (C₁ - C₁₂)alkyl
- -NRₐR_{b}, -NRₐC(=O)R_{b}, -NRₐC(=O)NRₐR_{b}, -NRₐC(=O)OR_{b}, -NRₐSO₂R_{b}, -ORₐ, -C(=O)Rₐ, -C(=O)ORₐ, -C(=O)NRₐR_{b}, -OC(=O)Rₐ, -OC(=O)NRₐR_{b}, -OC(=O)ORₐ, -SRₐ,-S(=O)Rₐ, -S(=O)₂Rₐ, -OS(=O)₂Rₐ, -S(=O)₂ORₐ, wherein Rₐ and R_{b} are independently selected from the group comprising hydrogen, (C₁ - C₁₂)alkyl, (C₁-C₁₂)alkenyl, (C₁-C₁₂)alkynyl, aryl, arylalkyl, heteroaryl, heteroarylalkyl, heterocycle, heterocyclealkyl, hydroxyl, halo, nitro, cyano, and halo (C₁ - C₁₂)alkyl;
wherein each of R₁, R₂, R₃, R₄, R₅ and R₆ may be optionally substituted with one or more substituents independently selected from the group comprising
- (C₁ - C₁₂)alkyl
- (C₂ - C₁₂)alkenyl,
- (C₂ - C₁₂)alkynyl,
- aryl,
- heteroaryl,
- aryl (C₁ - C₁₂)alkyl,
- heteroarylalkyl,
- heterocycle,
- heterocyclealkyl,
- halo,
- hydroxyl,
- nitro,
- cyano,
- halo (C₁ - C₁₂)alkyl
- -NRₐR_{b}, -NRₐC(=O)R_{b}, -NRₐC(=O)NRₐR_{b}, -NRₐC(=O)OR_{b}, -NRₐSO₂R_{b}, -ORₐ, -C(=O)Rₐ, -C(=O)ORₐ, -C(=O)NRₐR_{b}, -OC(=O)Rₐ, -OC(=O)NRₐR_{b}, -OC(=O)ORₐ, -SRₐ,-S(=O)Ra, -S(=O)₂Rₐ, -OS(=O)₂Rₐ, -S(=O)₂ORₐ, wherein Rₐ and R_{b} are independently selected from the group comprising hydrogen, (C₁ - C₁₂)alkyl, (C₁-C₁₂)alkenyl, (C₁-C₁₂)alkynyl, aryl, arylalkyl, heteroaryl, heteroarylalkyl, heterocycle, heterocyclealkyl, hydroxyl, halo, nitro, cyano, and halo (C₁ - C₁₂)alkyl
and
R₁, R₂, R₃, R₄, R₅ and R₆ are independently selected from:
- hydrogen,
- halo,
- hydroxyl,
- nitro, and
- cyano;

R₇ is selected from halo, halo (C₁ - C₁₂)alkyl, hydroxyl, -NRₐR_{b}, -NRₐC(=O)R_{b}, -NRₐC(=O)NRₐR_{b}, -NRₐC(=O)OR_{b}, -ORₐ, -C(=O)Rₐ, -C(=O)ORₐ, -C(=O)NRₐR_{b}, -OC(=O)Rₐ, -OC(=O)NRₐR_{b}, -OC(=O)ORₐ, wherein Rₐ and R_{b} are independently selected from the group comprising hydrogen, (C₁-C₁₂)alkyl, (C₁-C₁₂)alkenyl, (C₁-C₁₂)alkynyl, aryl, aryl (C₁ - C₁₂)alkyl, heteroaryl, heteroarylalkyl, heterocycle, heterocyclealkyl, hydroxyl, halo, nitro, cyano, halo (C₁ - C₁₂)alkyl; and

X is selected from -O-, -S-, or -NRₐ where Rₐ is defined as above. Preferably Rₐ is hydrogen.

In one embodiment R₁, R₂, R₃, R₄, R₅ and R₆ are independently selected from the group comprising
- hydrogen,
- (C₁ - C₁₂)alkyl
- (C₁ - C₁₂)alkenyl,
- (C₁ - C₁₂)alkynyl,
- aryl,
- heteroaryl,
- aryl (C₁ - C₁₂)alkyl,
- halo,
- hydroxyl,
- halo (C₁ - C₁₂)alkyl
- -NRₐR_{b}, -NRₐC(=O)R_{b}, -NRₐC(=O)OR_{b}, -ORₐ, -C(=O)Rₐ, -C(=O)ORₐ, -C(=O)NRₐR_{b}, -OC(=O)Rₐ, -OC(=O)NRₐR_{b}, -OC(=O)ORₐ, wherein Rₐ and R_{b} are independently selected from the group comprising hydrogen, (C₁ - C₁₂)alkyl, aryl, arylalkyl, hydroxyl, halo, and halo (C₁ - C₁₂)alkyl.

Preferably, R₁, R₂, R₃, R₄, R₅ and R₆ are independently selected from
- hydrogen,
- (C₁ - C₈)alkyl
- (C₂-C₈)alkenyl
- (C₂-C₈)alkynyl
- aryl,
- halo,
- hydroxyl,
- halo (C₁ - C₈)alkyl
- -NRₐR_{b}, -ORₐ, -C(=O)Rₐ, -C(=O)ORₐ, -OC(=O)Rₐ, -C(=O)NRₐR_{b}, -OC(=O)NRₐR_{b}, wherein Rₐ and R_{b} are independently selected from the group comprising hydrogen, (C₁ - C₆)alkyl, aryl, arylalkyl, hydroxyl, halo, and halo (C₁ - C₆)alkyl.

More preferably, R₁, R₂, R₃, R₄, R₅ and R₆ are independently selected from hydrogen, halo, halo (C₁-C₆)alkyl, (C₁ - C₅)alkyl, and (C₂-C₅)alkene, preferably (C₁-C₅)alkyl and hydrogen.

In one embodiment, R₁, R₂, R₄ and R₅ are methyl, R₃ is hydrogen, and R₆ is isopropyl.

In one embodiment R₇ is selected from the group comprising hydroxyl, -ORₐ, -OC(=O)Rₐ, -OC(=O)NRₐR_{b}, -OC(=O)ORₐ, wherein Rₐ and R_{b} are independently selected from the group comprising hydrogen, (C₁ - C₈)alkyl, (C₂-C₈)alkenyl, aryl, aryl (C₁ - C₈)alkyl, hydroxyl, halo, and halo (C₁ - C₈)alkyl.

Preferably, R₇ is selected from the group comprising hydroxyl, -ORₐ, and -OC(=O)Rₐ, wherein Rₐ is (C₁ - C₈)alkyl. More preferably, Rₐ is methyl.

In another preferred embodiment R₇ is -OC(=O)R_{c} where R_{c} is methyl, ethyl or propyl preferably methyl.

In a preferred embodiment X is O.

In one embodiment R₁, R₂, R₃, R₄, R₅ and R₆ are independently selected from hydrogen, halo, halo (C₁-C₅)alkyl, (C₁-C₅)alkyl and (C₁-C₅)alkenyl; R₇ is selected from the group comprising hydroxyl, -ORₐ, -OC(=O)Rₐ, -OC(=O)NRₐR_{b}, and -OC(=O)ORₐ, wherein Rₐ and R_{b} are independently selected from the group comprising hydrogen, (C₁ - C₈) alkyl, (C₂-C₈)alkene, aryl, aryl (C₁ - C₈)alkyl, hydroxyl, halo, and halo (C₁ - C₈)alkyl; and X is O.

Preferably, R₁, R₂, R₃, R₄, R₅ and R₆ are independently selected from hydrogen, halo (C₁-C₅)alkyl, (C₁-C₅)alkyl; R₇ is selected from the group comprising hydroxyl, -ORₐ, and -OC(=O)Rₐ, wherein Rₐ is (C₁-C₈)alkyl and X is O.

More preferably R₁, R₂, R₄, and R₅ are independently methyl or halomethyl, and R₃ is hydrogen, R₆ is (C₁-C₅)alkyl; R₇ is -OC(=O)Rₐ where Rₐ is (C₁-C₅)alkyl and X is O.

In a preferred embodiment X is NH.

In one embodiment R₁, R₂, R₃, R₄, R₅ and R₆ are independently selected from hydrogen, halo, halo (C₁-C₅)alkyl, (C₁-C₅)alkyl and (C₁-C₅)alkene; R₇ is selected from the group comprising hydroxyl, -ORₐ, -OC(=O)Rₐ, -OC(=O)NRₐR_{b}, and -OC(=O)ORₐ, wherein Rₐ and R_{b} are independently selected from the group comprising hydrogen, (C₁ - C₈) alkyl, (C₂-C₈)alkenyl, aryl, aryl (C₁ - C₈)alkyl, hydroxyl, halo, and halo (C₁ - C₈)alkyl; and X is NH.

Preferably, R₁, R₂, R₃, R₄, R₅ and R₆ are independently selected from hydrogen, halo (C₁-C₅)alkyl, (C₁-C₅)alkyl; R₇ is selected from the group comprising hydroxyl, -ORₐ, and -OC(=O)Rₐ, when Rₐ is (C₁-C₈)alkyl and X is NH.

More preferably R₁, R₂, R₄, and R₅ are methyl or halomethyl, and R₃ is hydrogen, R₆ is (C₁-C₅)alkyl; R₇ is -OC(=O)Rₐ where Rₐ is (C₁-C₅)alkyl and X is NH.

Preferably R₁, R₂, R₃, R₄ and R₅ are hydrogen, methyl or ethyl, R₆ is isopropyl, ethyl, isobutyl, or n-propyl and R₇ is ethoxy, methoxy, acetyloxy or propionyloxy, and X is NH.

In one embodiment the compound of formula (I) has (*R,R*) stereochemistry at the cyclopropyl carbon atoms.

In one aspect the invention provides a compound of formula (I) as defined above with the proviso that the compound is not (*1R*,*3R*)-2,-2-dimethyl-3-(2-methylprop-1-enyl)cyclopropyl)methyl (*2S*)-acetoxy-3-methylbutanoate.

In one embodiment the compound of formula (I) disclosed herein is a mealybug attractant, preferably a Citrophilous mealybug attractant.

In another aspect this disclosure provides a compound of formula II or a salt thereof 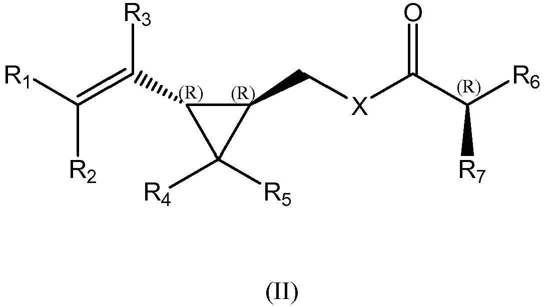 wherein R₁, R₂, R₃, R₄, R₅, R₆, R₇ and X are as defined above.

In one aspect the invention provides the compound (2,2-dimethyl-3-(2-methylprop-1-enyl)cyclopropyl)methyl 2-acetoxy-3-methylbutanoate (chrysanthemyl 2-acetoxy-3-methylbutanoate). Preferably the compound is ((*1R,3R*)-2,2-dimethyl-3-(2-methylprop-1-enyl)cyclopropyl)methyl (*2R*)-acetoxy-3-methylbutanoate. The invention is defined by the claims.

In another aspect this disclosure provides a method of synthesising the compound (2,2-dimethyl-3-(2-methylprop-1-enyl)cyclopropyl)methyl2-acetoxy-3-methylbutanoate, the method comprising:
(a) acetylating 2-hydroxy-3-methylbutanoic acid
(b) esterifying 2-acetoxy-3-methylbutanoic acid with chrysanthemol.

In one embodiment step (b) is carried out by converting 2-acetoxy-3-methylbutanoic acid to its acid halide and coupling the acid halide with chrysanthemol.

In another embodiment step (b) is carried out by actuating 2-acetoxy-3-methylbutanoic acid using a coupling agent such as DCC, and reacting the activated compound with chrysanthemol.

In one aspect the invention provides a Citrophilous mealybug attracting composition comprising (2,2-dimethyl-3-(2-methylprop-1-enyl)cyclopropyl)methyl2-acetoxy-3-methylbutanoate, preferably ((*1R,3R*)-2,2-dimethyl-3-(2-methylprop-1-enyl)cyclopropyl)methyl (*2R*)-acetoxy-3-methylbutanoate.

In one embodiment the Citrophilous mealybug attracting composition is formulated as a powder, granules, a paste, a liquid including a sprayable liquid or aerosol.

In one embodiment the Citrophilous mealybug attracting composition includes an insecticide. Preferably, the insecticide is one or more of buprofezin, pyriproxyfen, flonicamid, acetamiprid, dinotefuran, clothianidin, acephate, malathion, quinolphos, chloropyriphos, profenophos, bendiocarb, bifenthrin, chlorpyrifos, cyfluthrin, diazinon, fenpropathrin, kinoprene and/or insecticidal soap or oil.

In one aspect the invention provides a method of controlling or monitoring mealybug populations comprising providing a compound according to the claims in an amount effective to attract Citrophilous mealybugs.

In one embodiment mealybug populations are controlled by interfering with mealybug mating.

In another embodiment Citrophilous mealybug populations are controlled or monitored by attracting mealybugs to an area or object that includes a means of catching, immobilising and/or killing Citrophilous mealybugs.

In another aspect the invention provides a method of attracting Citrophilous mealybugs comprising providing a compound according to the claims in an amount effective to attract Citrophilous mealybugs.

In another aspect the invention provides a method of preventing damage to plants caused by Citrophilous mealybugs comprising attracting mealybugs away from the plants by providing a mealybug attractant according to the claims in an amount effective to attract Citrophilous mealybugs.

In the above aspects:
In one embodiment the compound is provided with one or more other mealybug attractants. Preferably the one or more mealybug attractants are mealybug pheromones.

In one embodiment the compound is provided in a mealybug attracting effective amount.

In one embodiment, the compound is provided with a carrier. Preferably the carrier includes a means of catching, immobilising and/or killing mealybugs. More preferably, the means of catching, immobilising and/or killing mealybugs comprises one or more insecticides.

In one embodiment of the compounds of this disclosure, R₁, R₂, R₃, R₄, R₅ and R₆ are independently selected from hydrogen, halo, halo (C₁-C₅)alkyl, (C₁-C₅)alkyl and (C₁-C₅)alkenyl; R₇ is selected from the group comprising hydroxyl, -ORₐ, -OC(=O)Rₐ, -OC(=O)NRₐR_{b}, and - OC(=O)ORₐ, wherein Rₐ and R_{b} are independently selected from the group comprising hydrogen, (C₁ - C₈) alkyl, (C₂-C₈)alkene, aryl, aryl (C₁ - C₈)alkyl, hydroxyl, halo, and halo (C₁ - C₈)alkyl; and X is O. In one embodiment R₁, R₂, R₃, R₄, R₅ and R₆ are independently selected from hydrogen, halo (C₁-C₅)alkyl, (C₁-C₅)alkyl; R₇ is selected from the group comprising hydroxyl, -ORₐ, and - OC(=O)Rₐ, wherein Rₐ is (C₁-C₈)alkyl and X is O.

In one embodiment of the compounds of this disclosure, R₁, R₂, R₄, and R₅ are independently methyl or halomethyl, R₃ is hydrogen, R₆ is (C₁-C₅)alkyl; R₇ is -OC(=O)Rₐ where Rₐ is (C₁-C₅)alkyl and X is O. In a preferred embodiment the compound of formula (I) is (2,2-dimethyl-3-(2-methylprop-1-enyl)cyclopropyl)methyl2-acetoxy-3-methylbutanoate, more preferably, the mealybug attractant is ((*1R,3R*)-2,2-dimethyl-3-(2-methylprop-1-enyl)cyclopropyl)methyl (*2R*)-acetoxy-3-methylbutanoate.

In one embodiment of this disclosure, the mealybug is selected from one or more of the Citrophilous mealybug, Madeira mealybug, Citrus mealybug, Pink Hibiscus mealybug I, Citriculus mealybug, Passionvine mealybug, Pink Hibiscus mealybug II, Comstock mealybug, Japanese mealybug, Vine mealybug, Longtailed mealybug, Grape mealybug and Obscure mealybug. Preferably, the mealybug is Citrophilous mealybug.

### 4. BRIEF DESCRIPTION OF THE FIGURES

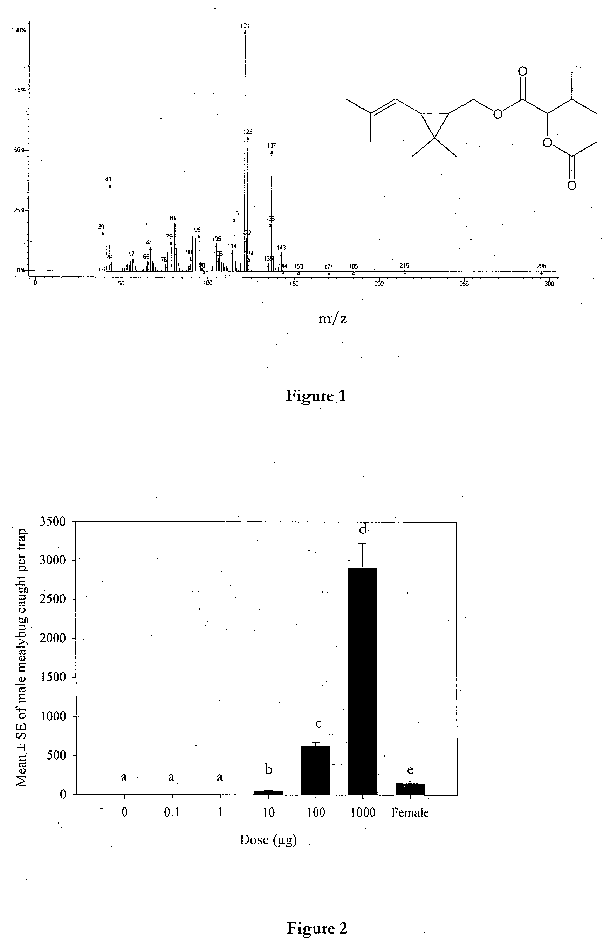

**Figure 1****.** EI mass spectrum (70eV) of chrysathemyl 2-acetoxy-3-methylbutanoate present in the headspace of female citrophilus mealybugs.
**Figure 2****.** Mean catch ± SE of male citrophilus mealybugs in red delta traps baited with various dosages of racemic chrysanthemyl 2-acetoxy-3-methylbutanoate (0.1, 1, 10, and 100 and 1000 µg) loaded on red rubber septa. Letters on columns indicate significant differences (FPLS Test, P *<* 0.05).

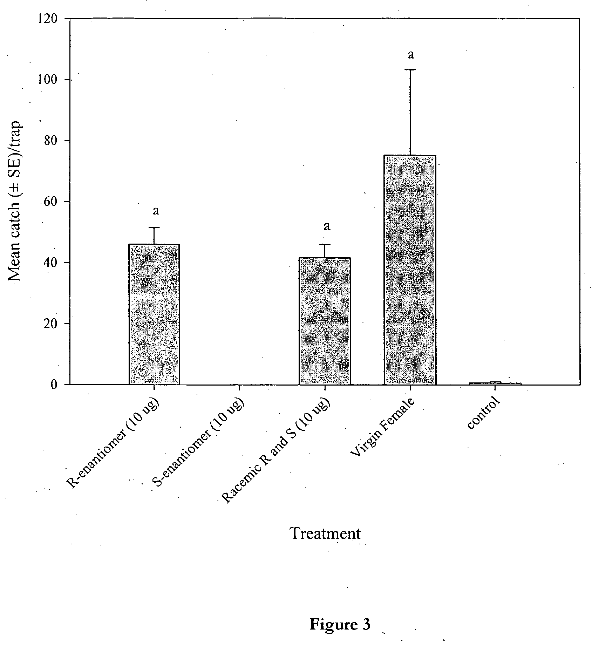

**Figure 3****.** Mean catch ± SE of male citrophilous mealybugs in red delta traps baited with a mixture of the four esters synthesized from *R*-acetate and cis/trans chrysanthemol (1:2), a mixture of the four esters synthesized from the *S*-acetate, a mixture of all eight esters, virgin females, and blank trap. Letters on columns indicate significant differences (FPLS Test, P < 0.05).

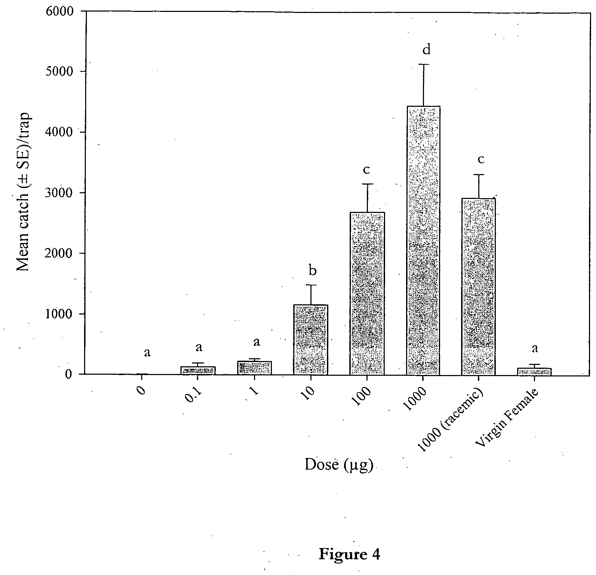

**Figure 4****.** Mean catch ± SE of male citrophilous mealybugs in red delta traps baited with various dosages of (2*R*)-acetoxy-3-methylbutanoate of (2*R*,3*R*)-chrysanthemol (0.1, 1, 10, and 100 and 1000 µg) loaded on red rubber septa. Letters on columns indicate significant differences (FPLS Test, P < 0.05).

### 5. DETAILED DESCRIPTION OF THE INVENTION

### 5.1 Definitions

The term "alkyl," as used herein means, unless otherwise stated, a straight or branched chain, cyclic or non-cyclic, fully saturated hydrocarbon radical, and may have the number of carbon atoms designated (i.e. C₁-C₁₀ means one to ten carbons). Where "alkyl" is a cyclic hydrocarbon radical, the radical may have up to the number of carbon atoms designated but must have at least three carbon atoms.

Examples of straight chain alkyl groups include methyl, ethyl, n-propyl, n-butyl, n-pentyl and n-hexyl. Examples of saturated branched chain alkyls include isopropyl, isobutyl, sec-butyl, test-butyl and isopentyl. Representative saturated cyclic alkyls include cyclopropyl, cyclobutyl, cyclopentyl, cyclohexyl and di-and poly-homocyclic rings such as decalin and adamentane.

The term "alkenyl" as used herein means, unless otherwise stated, a straight or branched chain, cyclic or non-cyclic, unsaturated hydrocarbon radical having at least one double bond. Examples of alkenyl groups include ethenyl, propenyl, 1-butenyl, 2-butenyl, 3-cyclohexenyl and the like.

The term "alkynyl" as used herein means, unless otherwise stated, a straight or branched chain, cyclic or non-cyclic, unsaturated hydrocarbon radical having at least one triple bond. Examples of alkynyl groups include ethynyl, propynyl, 1-butynyl and 2-butynyl.

The term "alkylene" as used herein means, unless otherwise stated, a divalent radical derived from an alkane, as exemplified by -CH₂CH₂CH₂CH₂-. Typically, an alkylene group will have from 1 to 24 carbon atoms.

The term "aryl," alone or in combination with other terms (e.g., aryloxy, arylalkyl) as used herein means, unless otherwise stated, an aromatic carbocyclic moiety that can be a single ring or multiple rings (for example, three rings) that are fused together or linked covalently, for example phenyl, 1-naphthyl, 2-naphthyl and 4-biphenyl.

The term "heteroaryl," as used herein means, unless otherwise stated, an aromatic ring of 5 to 10 members containing at least one heteroatom selected from nitrogen, oxygen and sulfur, and containing at least 1 carbon atom, including both mono- and bicyclic ring systems. The nitrogen and sulfur atoms are optionally oxidized, and the nitrogen atom(s) are optionally quaternized. The heteroaryl group may be attached to the parent moiety through a heteroatom or carbon atom.

Representative heteroaryl groups include (but are not limited to) furyl, benzofuranyl, thiophenyl, benzothiophenyl, pyrrolyl, indolyl, isoindolyl, azaindolyl, pyridyl, quinolinyl, isoquinolinyl, oxazolyl, isooxazolyl, benzoxazolyl, pyrazolyl, imidazolyl, benzimidazolyl, thiazolyl, benzothioazolyl, isothiazolyl, pyridazinyl, pyrimidinyl, pyrazinyl, triazinyl, cinnolinyl, phthalazinyl and quinazolinyl.

The term "arylalkyl" as used herein means, unless otherwise stated, an alkyl group having at least one hydrogen atom replaced with an aryl moiety such as a phenyl or naphthyl group including mono-, di-, and poly-homocyclic aromatic ring systems, for example, (C₆₋₁₄ aryl). Non-limiting examples of suitable arylalkyl groups include benzyl, phenylhexyl, 2-phenylethyl and naphthalenylmethyl. The bond to the parent moiety is through the alkyl group.

The term "heteroarylalkyl," as used herein means, unless otherwise stated, an alkyl group having at least one alkyl hydrogen atom replaced with a heteroaryl moiety, for example, -CH₂pyridinyl and -CH₂pyrimidinyl. The bond to the parent moiety is through the alkyl group.

The terms "heterocycle" and "heterocycle ring," as used herein, mean, unless otherwise stated, a 3-to 7-membered monocyclic, or 6- to 14-membered polycyclic, heterocycle ring which is either saturated, unsaturated or aromatic, and which contains from 1 to 4 heteroatoms independently selected from nitrogen, oxygen and sulphur, and wherein the nitrogen and sulphur heteroatoms may be optionally oxidized, and the nitrogen heteroatom may be optionally quarternized, including bicyclic rings in which any of the above heterocycles are fused to a benzene ring as well as tricyclic (and higher) heterocyclic rings. The heterocycle may be attached to the parent moiety via any heteroatom or carbon atom. Heterocycles include heteroaryls as defined above. Thus, in addition to the aromatic heteroaryls listed above, heterocycles also include (but are not limited to) morpholinyl, pyrrolidinonyl, pyrrolidinyl, piperidinyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl and tetrahydrothiopyranyl.

The term "heterocyclealkyl", as used herein means, unless otherwise stated, an alkyl having at least one alkyl hydrogen atom replaced with a heterocycle, for example -CH₂morpholinyl.

The terms "halo" or "halogen," as used herein mean, unless otherwise stated, a fluorine, chlorine, bromine, or iodine atom.

The term "haloalkyl" as used herein means, unless otherwise stated, an alkyl group having at least one hydrogen atom replaced with a halogen, for example monofluoroalkyl and trifluoromethyl.

The term "substituted" as used herein with reference to any of the above defined groups (e.g. alkyl, aryl, arylalkyl, heteroaryl, etc.) means a group or compound wherein at least one hydrogen atom has been replaced by a chemical substituent.

The term "comprising" as used herein means "consisting at least in part of". When interpreting each statement in this specification that includes the term "comprising", features other than that or those prefaced by the term may also be present. Related terms such as "comprise" and "comprises" are to be interpreted in the same manner.

The term "mealybug attractant" as used herein refers to a compound or composition that is capable of attracting at least one species of mealybug. A mealybug attractant may be selective for one species of mealybug only or attract more than one species of mealybug. Its attractant properties may vary with respect to individual species of mealybug depending on its concentration, the nature of the carrier material and/or manner in which it is provided.

A certain compound may exist in one or more particular geometric, optical, enantiomeric, diasteriomeric, epimeric, stereoisomeric, tautomeric, conformational, or anomeric forms, including but not limited to, cis- and trans-forms; E- and Z-forms; c-, t-, and r- forms; endo- and exo-forms; R-, S-, and meso-forms; D- and L-forms; (+) and (-) forms; keto-, enol-, and enolate-forms; α- and β-forms; axial and equatorial forms; boat-, chair-, twist-, envelope-, and halfchair-forms; and combinations thereof, hereinafter collectively referred to as "isomers" (or "isomeric forms").

The compounds of the invention have at least two asymmetrical carbon atoms and therefore all possible isomers, including enantiomers, stereoisomers, rotamers, tautomers and racemates of the compounds are contemplated as being part of this invention, unless otherwise stated. The invention includes d and l isomers in both pure form and in admixture, including racemic mixtures. Isomers can be prepared using conventional techniques, either by reacting optically pure or optically enriched starting materials or by separating isomers of a compound of the invention. Isomers may also include geometric isomers, e.g., when a double bond is present.

Note that, except as discussed below for tautomeric forms, specifically excluded from the term "isomers," as used herein, are structural (or constitutional) isomers (i.e., isomers which differ in the connections between atoms rather than merely by the position of atoms in space). For example, a reference to a methoxy group, -OCH₃, is not to be construed as a reference to its structural isomer, a hydroxymethyl group, -CH₂OH. Similarly, a reference to ortho-chlorophenyl is not to be construed as a reference to its structural isomer, meta-chlorophenyl. However, a preference to a class of structures may well include structurally isomeric forms falling within that class (e.g., C₁₋₇alkyl includes *n*-propyl and iso-propyl; butyl includes *n-, iso-, sec-,* and *tert*-butyl; methoxyphenyl includes *ortho-, meta-,* and *para*-methoxyphenyl).

The above exclusion does not pertain to tautomeric forms, for example, keto-, enol-, and enolate-forms, as in, for example, the following tautomeric pairs: keto/enol, imine/enamine, amide/imino alcohol, amidine/amidine nitroso/oxime, thioketone/enethiol, N- nitroso/hydroxyazo, and nitro / aci-nitro.

Unless otherwise specified, a reference to a particular compound includes all such isomeric forms, including racemic and other mixtures thereof. Methods for the preparation (e.g., asymmetric synthesis) and separation (e.g., fractional crystallisation and chromatographic means) of such isomeric forms are either known in the art or are readily obtained by adapting the methods taught herein in a known manner.

Unless otherwise specified, a reference to a particular compound also includes ionic, salt, hydrate, and protected forms of thereof, for example, as discussed below.

If the compound is cationic, or has a functional group which may be cationic (e.g., -NH may be -NH₃⁺), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous. Examples of suitable organic anions include, but are not limited to, anions from the following organic acids: acetic, propionic, succinic, gycolic, stearic, lactic, malic, tartaric, citric, ascorbic, maleic, hydroxymaleic, phenylacetic, glutamic, benzoic, salicylic, sulfanilic, 2-acetyoxybenzoic, fumaric, toluenesulfonic, methanesulfonic, ethane disulfonic, oxalic, isethionic, and valeric.

It may be convenient or desirable to prepare, purify, and/or handle a corresponding solvate of the active compound. The term "solvate" is used herein in the conventional sense to refer to a complex of solute (e.g., active compound, salt of active compound) and solvent. If the solvent is water, the solvate may be conveniently referred to as a hydrate, for example, a mono-hydrate, a dihydrate, a tri-hydrate, etc.

It may be convenient or desirable to prepare, purify, and/or handle the active compound in a chemically protected form. The term "chemically protected form," as used herein, pertains to a compound in which one or more reactive functional groups are protected from undesirable chemical reactions, that is, are in the form of a protected or protecting group (also known as masked or masking group). By protecting a reactive functional group, reactions involving other unprotected reactive functional groups can be performed, without affecting the protected group; the protecting group may be removed, usually in a subsequent step, without substantially affecting the remainder of the molecule. See, for example, Protective Groups in Organic Synthesis (P. G. M Wuts, T. W. Greene; Greene's Protective Groups in Organic Synthesis 4th ed., Wiley 2007).

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

### 5.2 Compounds of this disclosure

The sex pheromone of a citrophilus mealybug was identified from the headspace of virgin females as chrysanthemyl 2-acetoxy-3-methylbutanoate using GC-MS analysis, derivatisation procedures and field trapping experiments with a synthetic version of the pheromone. Figure 1 shows the EI mass spectrum (70eV) of chrysanthemyl 2 acetoxy-3-methylbutanoate. The absolute configuration determined by NMR, derivatisation reactions and chiral GC-MS analysis with synthetic chiral reference compounds was determined to be the (2*R*)-acetoxy-3-methylbutanoate of (*2R,3R*)-chrysanthemol.

An isomeric mixture of synthetic chrysanthemyl 2-acetoxy-3-methylbutanoate proved to be highly attractive to male mealybugs as outlined in Examples 2 and 3.

Racemic chrysanthemyl 2-acetoxy-3-methoxybutanoate can be readily synthesised from commercially available intermediates as per Scheme 1 below.

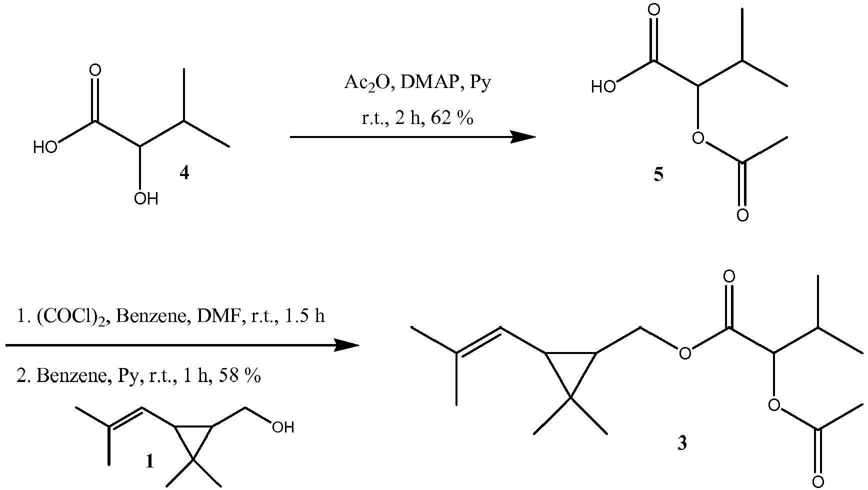

2-hydroxy-3-methylbutanoic acid (4) is acetylated with acetic anhydride to provide (5). 2-acetoxy-3-methylbutanoic acid (5) is then converted to the acid halide using oxalyl chloride then esterified with chrysanthemol.

In an alternate process, dicyclocarbodiimide (DCC) is used to couple the unactivated carboxylic acid.

The details are outlined in Example 1.

Similar synthetic techniques known in the art can be used to obtain other compounds of formula (I). For example, (5) can be replaced by a variety of 2-substituted carboxylic acids to alter the substitutents at the R6 and R7 positions. Replacement of the alcohol unit in chrysanthemol with an amine derivative will result in amide linked compounds of formula (I). The isoprene unit of chrysanthemol can be easily halogenated to produce halo or haloalkyl substitutents and halogenated sites may act as effective leaving groups that can be substituted with other groups. A person skilled in the art would be able to synthesise a range of compounds within the scope of formula (I), as defined above.

Stereoisomerically pure compounds may be achieved by the use of chiral starting materials, in combination with standard separation techniques.

The mealybug attracting properties of the compounds of formula (I) may vary in respect to the species of mealybug they are being tested against.

It is possible for a compound of formula (I) to attract one or more species of mealybug but be inactive towards other species. Compounds of formula (I) found to be inactive towards certain species of mealybug may be attractants for other untested mealybug species.

The mealybug attractant properties of a compound of formula (I) can be assessed using techniques known in the art. For example, insect attraction can be assessed using a glass Y-tube olfactomer following the method described by de Kogel et al. (de Kogel, W. J., Koschier, E. H. and Visser, J. H. Proc. Exper. & Appl. Entomol., N. E. V. Amsterdam, (1999) 10, 131-135.) and Koschier et al. (Koschier, E. H., De Kogel, W. J. and Visser, J. H. (2000) Journal of Chemical Ecology, 26, 2643-2655).

Activity may also be assessed in field trials utilising traps containing the potential mealybug attracting compound. An example of a field trial is described in Example 2.

### 5.3 Mealybug attracting compositions of this disclosure

The mealybug attracting compounds of formula (I) can be used to prepare a mealybug attracting composition for use in the methods of the invention.

In one aspect the invention provides a mealybug attracting composition comprising a compound of formula (I) in an amount effective to attract mealybugs and a carrier material.

The carrier material may be a liquid, gaseous or solid material and may be integral or non-integral with the mealybug attractant. Non-integral carriers include but are not limited to traps and lures that contain the attractant compound and allow for its release.

In one aspect the invention provides a trap comprising a mealybug attracting composition of formula (I). Integral carriers are mixed with the attractant to form a mealybug attracting material. Examples of solid carriers include but are not limited to fillers such as kaolin, bentonite, dolomite, calcium carbonate, talc, powdered magnesia, Fuller's earth, gypsum, diatomaceous earth and China clay. Examples of liquid carriers include but are not limited to water; alcohols, particularly butanol or glycol, as well as their ethers or esters, particularly methylglycol acetate; ketones, particularly acetone, cyclohexanone, methylethyl ketone, methylisobutylketone, or isophorone; petroleum fractions such as paraffinic or aromatic hydrocarbons, particularly xylenes or alkyl naphthalenes; mineral or vegetable oils; aliphatic chlorinated hydrocarbons, particularly trichloroethane or methylene chloride; aromatic chlorinated hydrocarbons, particularly chlorobenzenes; water-soluble or strongly polar solvents such as dimethylformamide, dimethyl sulfoxide, or *N*-methylpyrrolidone; liquefied gases; or the like or a mixture thereof.

A carrier which provides for slow or delayed release of a mealybug attractant of the invention may also be included in the composition. Carriers may also include materials and devices such as bubble caps comprising a reservoir with a permeable barrier through which attractants are slowly released, attractant-impregnated synthetic polymers in various shapes, such as pellets, granules and ropes, attractant-impregnated inert natural materials such as clays, cellulose and rubber. It is to be understood that a skilled worker will be able, without undue experimentation, with regard to that skill and this disclosure, to select a suitable carrier for the desired mode of application, storage, transport or handling.

The mealybug attractant compound of formula (I) may be provided in combination with other attractants provided that the other compounds do not substantially interfere with the activity of the compound of formula (I). The other attractants may be other compounds of formula (I) or may be other known mealybug pheromone compounds of different structure. Many mealybug pheromones have been discovered and identified, for example, the pheromones used by citriculus mealybug, vine mealybug, grape mealybug, obscure mealybug and long-tailed mealybug. Such pheromones can be combined with the compounds of formula (I) to target populations of different mealybug species. The compound of formula (I) may also be combined with non-pheromone attractant compounds.

The mealybug attractant compositions of the invention may also include insecticide. Examples of insecticides that may be used in combination with the attractants of formula (I) include, but are not limited to buprofezin, pyriproxyfen, flonicamid, acetamiprid, dinotefuran, clothianidin, acephate, malathion, quinolphos, chloropyriphos, profenophos, bendiocarb, bifenthrin, chlorpyrifos, cyfluthrin, diazinon, fenpropathrin, kinoprene and/or insecticidal soap or oil.

As will be apparent to one of skill in the art, the amount and/or concentration of compound of formula (I) required for a particular application may vary depending on several factors such as (1) the nature of the subject, (2) the physiological condition of the subject, (3) the type and level of infestation, (4) the type of composition used, (5) the type of wound on the subject, and (6) environmental factors such as temperature and humidity. It should be understood that a person of ordinary skill in the art will be able, without undue experimentation, having regard to that skill and this disclosure, to determine an effective amount of a composition of this invention for a given application.

In one embodiment the compositions of the invention may be formulated to comprise about 0.001 to 95% by weight of one or more compounds of the invention, preferably 0.001 to 90% by weight, more preferably 0.001 to 85% by weight, more preferably 0.001 to 75% by weight, more preferably 0.001 to 75% by weight, more preferable 0.001 to 70% by weight, more preferably 0.001 to 65% by weight, more preferably 0.001 to 60% by weight, more preferably 0.001 to 55% by weight, more preferably 0.001 to 50% by weight, more preferably 0.001 to 45% by weight, more preferably 0.001 to 40% by weight, more preferably 0.001 to 35% by weight, more preferably 0.001 to 30% by weight, more preferably 0.001 to 25% by weight, more preferably 0.001 to 20% by weight, more preferably 0.001 to 15% by weight, more preferably 0.001 to 10% by weight, more preferably 0.005 to 10% by weight, more preferably 0.01 to 10% by weight, more preferably 0.1 to 10% by weight, more preferably 0.5 to 10% by weight, more preferably 1.0 to 10% by weight more preferably 2 to 8% by weight, more preferably 4 to 6% by weight and useful ranges may be selected between any of the foregoing values, for example, 10 to 20% by weight.

### 5.4 Methods of the invention

The mealybug attracting compounds of the invention can be used to control or monitor Citrophilous mealybugs.

In the methods of the invention, the mealybug attractant compound of claim 1 can be provided by any means known in the art. For example, the compound may be provided in a carrier that allows emission of the compound. Examples of such carriers include but are not limited to pads, beads, rods, spirals or balls composed of rubber, plastic, leather, cotton, cotton wool, wood or wood products that are impregnated with the attractant. Carriers are described in detail above.

The carrier may also contain a bait or feeding stimulant that will entice the mealybug to ingest an effective amount of the insecticide.

In one embodiment, the mealybug attractant compound of claim 1 may be mixed in a paste that can be applied to trees and other plants. Insecticides added to the paste will kill mealybugs that contact the paste.

The mealybug attractant compound of claim 1 may also be applied directly or with an integral carrier such as a solvent or gas to surfaces in the area of a population of mealybugs. Such surfaces include the ground surface surrounding the plants, the surface of the plants themselves, walls or structures in the vicinity of the plants, planting equipment, harvesting and packaging equipment and the like.

The carrier may also comprise a trap.

The trap is positioned in an area infested (or potentially infested) with mealybugs. The aroma of the attractant attracts mealybugs to the trap. The insects may then be caught, immobilised and/or killed within the trap, for example, by the insecticide present in the trap. For example, the mealybug attractant of formula (I) may be applied to an absorbent material attached to the sticky surface of a sticky trap.

It will be appreciated by a person skilled in the art that a variety of different carrier traps are possible. Suitable examples of such traps include water traps, sticky traps, and one-way traps. Sticky traps come in many varieties. One example of a sticky trap is of cardboard construction, triangular or wedge-shaped in cross-section, where the interior surfaces are coated with a non-drying sticky substance. The insects contact the sticky surface and are caught. Sticky traps are available from Olson Products, Medina, Ohio, USA and come in a range of different colours.

Water traps include pans of water and detergent that are used to trap insects. The detergent destroys the surface tension of the water, causing insects that are attracted to the pan, to drown in the water. One way traps allow an insect to enter the trap but prevent it from exiting.

The traps of the invention may be coloured brightly, to provide additional attraction for mealybugs.

The amount of mealybug attractant compound provided will be at least an effective amount. An effective amount is the minimum amount of attractant compound needed to attract mealybugs to a treated area or object when compared to the same untreated area or object. The precise amount needed to be effective will vary with the particular compound of formula (I) or combination of compounds, the type of area or object treated, the length of time the method is to be used and the environment in which the area or object is located. This precise amount can easily be determined by one skilled in the art.

Mealybug populations can be controlled by using the methods of the invention to remove the population away from a target area, or by using the methods of the invention in conjunction with traps or insecticides for catching, immobilising or killing the psyllids.

Mealybug populations can also be surveyed or monitored by counting the number of insects caught. Based on the estimated population, decisions can be made regarding the need for population control. For example, a discovery of a high mealybug population may necessitate the use of methods for removal of the mealybug, such as provided by the methods of the invention. Conversely, a discovery of a low mealybug population may lead to a decision that it is sufficient to continue monitoring the population. Mealybug populations may be monitored regularly so that populations are controlled only when they reach a certain threshold. This provides cost-effective control of the insects and reduces the environmental impact of the use of insecticides. Populations can be monitored by providing mealybug attractant compounds of claim 1 in a trap that can immobilise the insects so that numbers can be inspected by a horticulturist.

Knowing where insects are, how many of them there are, and their life stage enables informed decisions to be made as to where and when insecticides or other treatments are warranted.

The following non-limiting examples are provided to illustrate the present invention and in no way limits the scope thereof.

### 6. EXAMPLES

### Example 1: Synthesis of racemic chrysanthemyl 2-acetoxy-3-methylbutanoate

334 mg (2.82 mmol) 2-hydroxy-3-methylbutanoic acid was dissolved together with a catalytic amount of 4-dimethylaminopyridine (DMAP) in 10 mL of dry pyridine. Acetic anhydride (2.00 mL; 21.2 mmol) was added and the solution was stirred for 2 h at room temperature. After the addition of water, the mixture was acidified to pH 3 by addition of 2M HCl and extracted twice with diethyl ether. The combined organic phases were washed with water and brine, dried over MgSO₄ and concentrated to yield 280 mg (62 %) of a colorless oil. 250 mg (1.56 mmol) of the crude 2-acetoxy-3-methylbutanoic acid was dissolved in 1.5 mL benzene and 0.22 mL (2.5 mmol) oxalyl chloride and 1 µL DMF were added. After 1.5 h stirring at room temperature, the solvent and excess reagent were removed by evaporation. The residue was dissolved in 1.5 mL benzene and a solution of 0.3 mL (1.66 mmol) chrysanthemol (Aldrich, 65:35 trans/cis mixture of isomers) and 0.2 mL pyridine in 1.5 mL benzene was added dropwise. After 1 h of stirring at room temperature, the solvent was evaporated and water was added. The mixture was extracted twice with diethyl ether, the combined organic layers were washed with water, 1M HCl, and brine, dried over MgSO₄, and concentrated. The crude product was purified by column chromatography on silica (10 % ethyl acetate in hexane) to yield 270 mg (58 %) of pure **3**.¹HNMR (400 MHz, CDCl₃): δ = 4.92-4.86 (m, 0.3H), 4.85-4.79 (m, 1.7H), 4.45 (dd, 0.3H, J = 6.8, 11.7 Hz), 4.31 (dd, 0.3H, J = 6.6, 11.7 Hz), 4.23 (dd, 0.2H, J = 7.6, 11.7 Hz), 4.13 (dd, 0.3H, J = 6.6, 7.6 Hz), 4.05 (dd, 0.5H, j = 9.0, 11.8 Hz), 3.94 (dd, 0.3H, J = 9.0, 11.7 Hz), 2.26-2.16 (m, 0.8H), 2.12 (s, 3H), 1.69 (s, 3H), 1.65 (s, 3H), 1.40 (t, 0.3H, J = 8.3 Hz), 1.17 (dd, 0.7H, J = 5.2, 7.6 Hz), 1.13-0.94 (m, 12.5H), 0.91-0.84 (m, 0.8H) ppm. 13C-NMR (CDCl₃, 100.6 MHz): δ = 170.68, 169.92, 169.78, 135.86, 133.66, 122.72, 118.46, 118.42, 76.87, 76.84, 66.34, 66.29, 63.90, 63.85, 30.82, 30.73, 30.02, 29.14, 28.91, 28.48, 26.42, 26.38, 26.36, 25.62, 25.58, 25.55, 22.27, 21.51, 21.44, 20.58, 18.65, 18.60, 18.47, 18.23, 17.22, 17.16, 15.49, 15.45 ppm., MS (70 eV) m/z: 43(100), 55(17), 57(12), 67(10), 81(17), 82(18), 93(16), 95(12), 115(13), 121(39), 123(28), 143(10), 296(<1, M+).

### Example 2: Field trial of racemic chrysanthenyl 2-acetoxy-3-methylbutanoate in New Zealand

Five doses of racemic chrysanthemyl 2-acetoxy-3-methylbutanoate (0.1, 1, 10, 100, and 1000 µg) loaded into red rubber septa were tested for the attraction of *P. calceolariae* in vineyards near Hawkes Bay, New Zealand for two weeks in February 2009 using a randomized complete block design. A septum was loaded with 0.1, 10, 100, and 1000 µg of chrysanthemyl 2-acetoxy-3-methylbutanoate dissolved in 200 µl hexane, and the solvent was allowed to evaporate in a fume hood. The septa were stored at -20 °C until ready for use. Traps were placed in five rows with five replications for each treatment at 15 m spacing between trapping stations and treatment rows. Septa were placed in the centre of the white sticky base lying on the sticky surface. Traps baited with virgin female were used as positive controls, while traps baited with blank lures were used as negative controls. One of each of the five treatments was randomly assigned to a trap tree within each row of trees. The significance in the quantity of the treatment effect in the field experiments were tested using ANOVA (Statview, SAS Institute Inc., Cary, North Carolina). Significantly different means were identified using Fisher's Protected Least Significant Difference.

The amount of chrysanthemyl 2-acetoxy-3-methylbutanoate significantly affected the number of captured males (Fig. 2). The highest number of males was captured with the 1000 µg loading. There was no difference in the mean numbers of male captured using the 0, 0.1, and 1 µg loading, while increasing the loading from 1 to 10 µg resulted in a significant increase in the mean number of captures. Traps baited with 100 and 1000 µg doses captured 4 to 20-fold more males compared to traps baited with virgin females respectively.

### Example 3: Field trial of racemic chrysanthenyl 2-acetoxy-3-methylbutanoate in Chile

In Chile, a field test was conducted from 3-7 march 2009 in a raspberry plantation (0.43 ha; 5714 plants/ha) near Nogales (Valparaíso Region, Central Chile) using a randomized complete block design. White rubber septa were loaded with a single 100 µg of chrysanthemyl 2-acetoxy-3-methylbutanoate in hexane (treatment) or hexane (control). The solvent was allowed to evaporate in a fume hood and septa were stored at 5 °C until use. Septa were placed in the centre on the sticky surface inside the delta traps. One control and one pheromone trap were placed in the same row, at 20 m spacing between traps, in four different rows (replicates) that were separated at least by 9 m. Males caught in traps were counted 48 hours after setting the experiment. 1171±270 males (n=4) were captured in the test traps while no males were captured in the control traps.

### Example 4: Synthesis of non-racemic chrysanthemyl 2-acetoxy-3-methylbutanoate

Four stereoisomeric pairs of chrysanthemyl 2-acetoxy-3-methylbutanoate were produced from a mixture of chrysanthemol isomers and (2*R*)-acetoxy-3-methylbutanoate or (2*S*)-acetoxy-3-methylbutanoate in separate vials as described in Example 1. The first synthetic step was an acylation of the chiral acids using acetic anhydride and the second step was a dicyclohexylcarbodiimide-mediated esterification between chrysanthemol and the chiral 2-acetoxy-3-methylbutanoic acids with DMAP (dimethylaminopyridine) as catalyst. The reaction produced four R-esters and four S-esters. The spectroscopic data was identical with the data reported in Example 1.

**Example 5: Synthesis of (*R,R,R*)-Chrysanthemyl 2-acetoxy-3-methylbutanoate.**

(*R,R,R*)-Chrysanthemyl 2-acetoxy-3-methylbutanoate was prepared from (2*R*)-hydroxy-3-methylbutanoic acid and acetic anhydride in analogy with the two step procedure described for the "racemic" chrysanthemyl 2-acetoxy-3-methylbutanoate in Example 4.

The spectroscopic data is provided below.

H NMR, (400 MHz, CDCl₃) δ: 4.86 (1H, dt J=7.8, 1.1 Hz), 4.84 (1H, d J=4.6 Hz), 4.48 (1H, dd J=11.7, 6.7 Hz), 3.97 (1H, dd J=11.7, 9.0 Hz), 2.24 (1H, dhept J=6.9, 4.6 Hz), 2.15 (3H, s), 1.71 (3H, s), 1.67 (3H, s), 1.19 (1H, dd J=7.8, 5.3 Hz), 1.15 (3H, s), 1.05 (3H, m), 1.02 (3H, d J=6.9 Hz), 1.00 (3H, d J=6.9 Hz), 0.90 (1H, ddd J=9.0, 6.7, 5.3 Hz) ppm.

¹³C NMR, (CDCl₃) 170.69, 169.81, 133.69, 122.74, 76.86, 66.36, 30.88, 30.05, 29.19, 25.56, 22.46, 22.29 21.54, 20.59, 18.63, 18.26, 17.26 ppm.

### Example 6: Field test of various steroisomers of chrysanthemyl 2-acetoxy-3-methylbutanoate

The activity of various isomers of (2,2-dimethyl-3-(2-methylprop-1-enyl)cyclopropyl)methyl 2-acetoxy-3-methylbutanoate produced in Example 4 were tested for the attraction of *P. calceolariae* in vineyards near Hawkes Bay, New Zealand for two weeks in Feburary 2009 using a randomized complete block design. The treatments included; 10 µg of a mixture of the four esters of (2*R*)-acetate and cis/trans chrysanthemol (1:2); 10 µg of (2*S*)-acetate cis/trans chrysanthemol; and 10 µg of all eight stereoisomers of chrysanthemyl 2-acetolxy-3-methylbutanoate. Virgin females were used as positive control and blank trap was used as negative control. Chemicals were dissolved in

200 µl hexane, and the solvent was allowed to evaporate in a fume hood. The septa were stored at - 20 °C until ready for use. Traps were placed in five rows with five replications for each treatment at 15 m spacing between trapping stations and treatment rows. Septa were placed in the centre of the white sticky base lying on the sticky surface. One of each of the treatments tested was randomly assigned to a trap tree within each row of trees. The significance in the quantity of the treatment effect in the field experiments were tested using ANOVA (Statview, SAS Institute Inc., Cary, North Carolina, USA (1998). Significantly different means were identified using Fisher's Protected Least Significant Difference.

The results are shown in Figure 3.

### Example 7: Dosage trial of (R,R,R)-chrysanthemyl 2-acetoxy-3-methylbutanoate

To investigate the effect of dosage on male catch, five doses of the (2*R*)-acetoxy-3-methylbutanoate of (2R,3R)-chrysanthemol (0, 0.1, 1, 10, 100, and 1000 µg) loaded into red rubber septa were tested for the attraction of *P. calceolariae* in the same location in vineyards near Hawkes Bay, New Zealand in March 2009. The experiment had a randomized complete block design and traps baited with virgin females were used as positive control.

Chemicals were dissolved in 200 µl hexane, and the solvent was allowed to evaporate in a fume hood. The septa were stored at -20 °C until ready for use. Traps were placed in five rows with five replications for each treatment at 15 m spacing between trapping stations and treatment rows. Septa were placed in the centre of the white sticky base lying on the sticky surface. One of each of the treatments tested was randomly assigned to a trap tree within each row of trees. The significance in the quantity of the treatment effect in the field experiments were tested using ANOVA (Statview, SAS Institute Inc., Cary, North Carolina, USA (1998). Significantly different means were identified using Fisher's Protected Least Significant Difference.

The results are shown in Figure 4. The amount of (2R)-acetoxy-3-methylbutanoate of (2*R*,3*R*)-chrysanthemol significantly affected the number of captured males. The highest number of males were captured with the 1000 µg loading, which caught 36-fold more males compared to traps baited with virgin females.

It is not the intention to limit the scope of the invention to the abovementioned examples only. As would be appreciated by a skilled person in the art, many variations are possible without departing from the scope of the invention (as set out in the accompanying claims).

### References

1) Petersen, C. L.; Charles, J. G. Plant Pathology 1997, 46, 509-515.
2) El-Sayed, A. M. http://www.pherobase.com **2008**.
3) Millar, J. G.; Daane, K. M.; Mc Elfresh, J. S.; Moreira, J. A.; Bentley, W. J. In Semiochemicals in Pest and Weed Control, Petroski, R. D.; Tellez, M. R.; Behle, R. W. Eds.;ACS Symposium Series 906; 2005; pp 11-27.15.
4) Rotundo, G.; Tremblay, E. J. Chem. Ecol. 1981, 7, 85-88.

## Claims

1. A compound selected from the group consisting of: 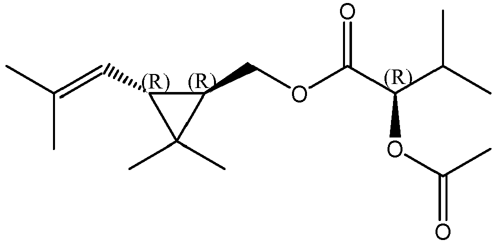 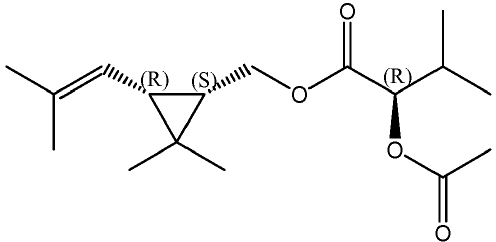 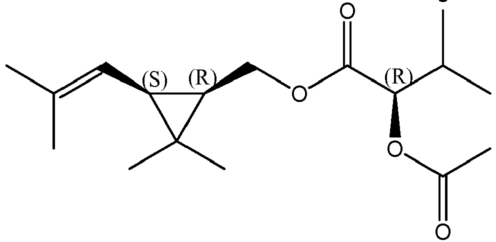 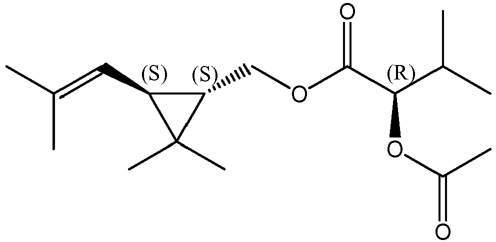 or a salt thereof.

2. A compound of claim 1, wherein the compounds is: 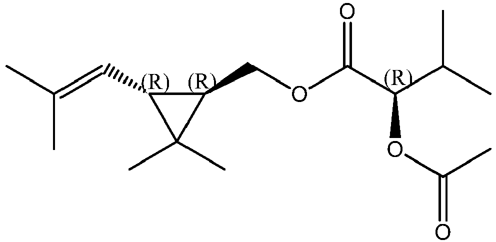 or a salt thereof.

3. A Citrophilous mealybug attracting composition comprising a compound as defined in claim 1 or claim 2 in an amount effective to attract Citrophilous mealybugs and a carrier material.

4. A Citrophilous mealybug attracting composition of claim 3 additionally comprising an insecticide.

5. A Citrophilous mealybug attracting composition of claim 3 or claim 4 wherein the composition is formulated as a powder, granules, a paste or a liquid including a sprayable liquid or aerosol.

6. A method of controlling or monitoring Citrophilous mealybug populations comprising providing a compound as defined in claim 1 or 2 in an amount effective to attract Citrophilous mealybugs.

7. A method according to claim 6 wherein the Citrophilous mealybug populations are controlled or monitored by attracting Citrophilous mealybugs to an area or object that includes a means of catching, immobilising and/or killing Citrophilous mealybugs.

8. A method according to claim 6 wherein the Citrophilous mealybug populations are controlled by interfering with Citrophilous mealybug mating.

9. A method of attracting Citrophilous mealybugs comprising providing a compound as defined in claim 1 or 2 in an amount effective to attract Citrophilous mealybugs.

10. A method of preventing damage to plants caused by Citrophilous mealybugs comprising attracting Citrophilous mealybugs away from the plants by providing a compound as defined in claim 1 or 2 in an amount effective to attract Citrophilous mealybugs.

## Patentansprüche

1. Verbindung, ausgewählt aus der aus den folgenden bestehenden Gruppe: 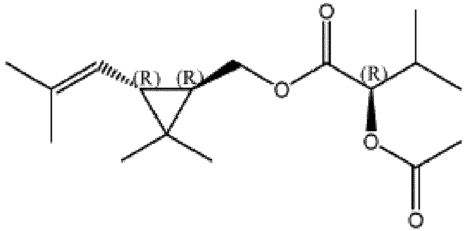 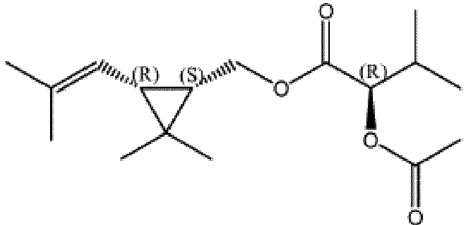 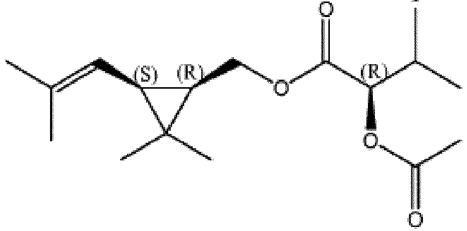 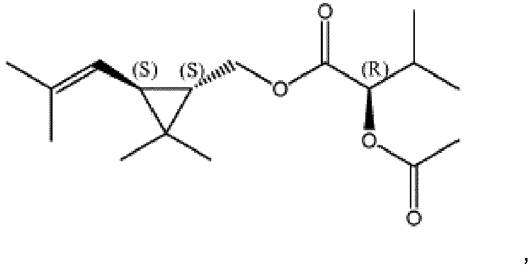 oder Salz davon.

2. Verbindung nach Anspruch 1, worin die Verbindung die folgende ist: 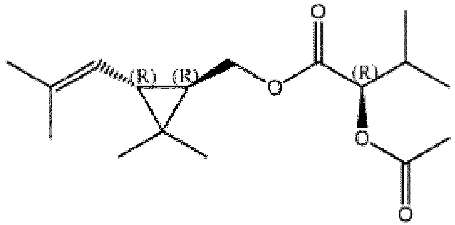 oder Salz davon.

3. Zusammensetzung zum Anlocken von Zitrusschmierläusen, die eine Verbindung nach Anspruch 1 oder Anspruch 2 in einer zum Anlocken von Zitrusschmierläusen wirksamen Menge sowie ein Trägermaterial umfasst.

4. Zusammensetzung zum Anlocken von Zitrusschmierläusen nach Anspruch 3, die zusätzlich ein Insektizid umfasst.

5. Zusammensetzung zum Anlocken von Zitrusschmierläusen nach Anspruch 3 oder Anspruch 4, worin die Zusammensetzung als Pulver, Granulat, Paste oder Flüssigkeit, einschließlich einer sprühbaren Flüssigkeit oder eines Aerosols, formuliert ist.

6. Verfahren zur Eindämmung oder Überwachung von Zitrusschmierlauspopulationen, welches das Bereitstellen einer Verbindung nach Anspruch 1 oder 2 in einer Menge umfasst, die zum Anlocken von Zitrusschmierläusen wirksam ist.

7. Verfahren nach Anspruch 6, worin die Zitrusschmierlauspopulationen dadurch eingedämmt oder überwacht werden, dass Zitrusschmierläuse in einen Bereich oder zu einem Objekt gelockt werden, der/das ein Mittel zum Einfangen, Immobilisieren und/oder Töten von Zitrusschmierläusen umfasst.

8. Verfahren nach Anspruch 6, worin die Zitrusschmierlauspopulationen durch Störung der Zitrusschmierlauspaarung eingedämmt werden.

9. Verfahren zum Anlocken von Zitrusschmierläusen, welches das Bereitstellen einer Verbindung nach Anspruch 1 oder 2 in einer Menge umfasst, die zum Anlocken von Zitrusschmierläusen wirksam ist.

10. Verfahren zum Verhindern von durch Zitrusschmierläuse verursachten Schäden an Pflanzen, welches das Weglocken von Zitrusschmierläusen von den Pflanzen umfasst, indem eine Verbindung nach Anspruch 1 oder 2 in einer Menge bereitgestellt wird, die zum Anlocken von Zitrusschmierläusen wirksam ist.

## Revendications

1. Composé choisi dans le groupe constitué de : 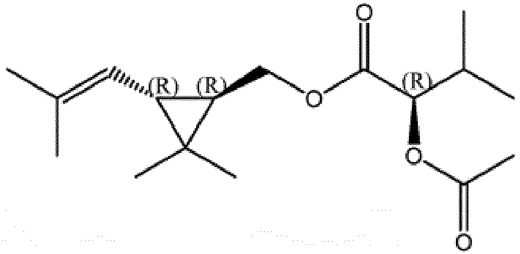 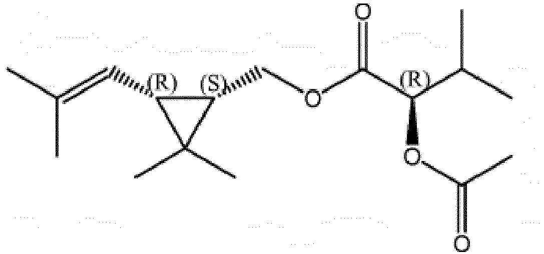 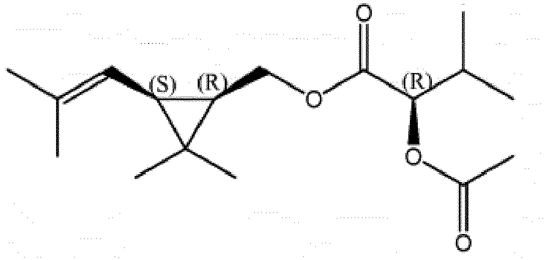 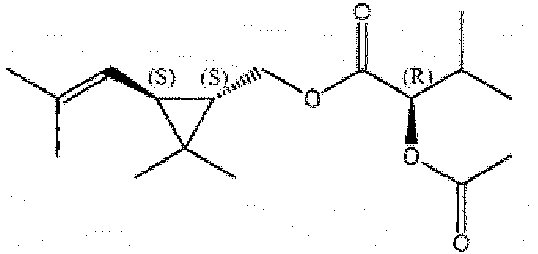 ou un sel de celui-ci.

2. Composé selon la revendication 1, dans lequel le composé est : 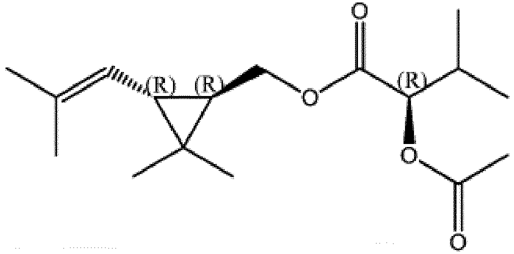 ou un sel de celui-ci.

3. Composition attirant la cochenille citrophile comprenant un composé tel que défini dans la revendication 1 ou la revendication 2 en une quantité efficace pour attirer les cochenilles citrophiles, et un matériau porteur.

4. Composition attirant la cochenille citrophile selon la revendication 3, comprenant en outre un insecticide.

5. Composition attirant la cochenille citrophile selon la revendication 3 ou la revendication 4, dans laquelle la composition est formulée sous la forme d'une poudre, de granules, d'une pâte ou d'un liquide comprenant un liquide pulvérisable ou un aérosol.

6. Procédé de contrôle ou de surveillance de populations de cochenilles citrophiles comprenant la fourniture d'un composé tel que défini dans la revendication 1 ou 2 en une quantité efficace pour attirer des cochenilles citrophiles.

7. Procédé selon la revendication 6, dans lequel les populations de cochenilles citrophiles sont contrôlées ou surveillées en attirant des cochenilles citrophiles vers une zone ou un objet qui comprend un moyen de capture, d'immobilisation et/ou de destruction des cochenilles citrophiles.

8. Procédé selon la revendication 6, dans lequel les populations de cochenilles citrophiles sont contrôlées en interférant avec l'accouplement des cochenilles citrophiles.

9. Procédé d'attraction de cochenilles citrophiles comprenant la fourniture d'un composé tel que défini dans la revendication 1 ou 2 en une quantité efficace pour attirer les cochenilles citrophiles.

10. Procédé de prévention de dommages causés à des plantes par des cochenilles citrophiles consistant à attirer les cochenilles citrophiles loin des plantes en fournissant un composé tel que défini dans la revendication 1 ou 2 en une quantité efficace pour attirer les cochenilles citrophiles.
